# FASCICULE DE BREVET EUROPEEN

(11) **EP 2 585 147 B1**
(45) Date de publication et mention de la délivrance du brevet: **31.10.2018**
(21) Numéro de dépôt: 11734002.6
(22) Date de dépôt: 28.06.2011
(51) Int. Cl.: A61M 39/02, A61M 5/158, A61M 5/32, A61M 25/06

(54) **AIGUILLE AVEC UN DISPOSITIF DE SECURITE**
NADEL MIT EINER SICHERHEITSVORRICHTUNG
NEEDLE HAVING A SAFETY DEVICE

(30) Priorité: 28.06.2010 FR 1002701
(43) Date de publication de la demande: 01.05.2013
(73) Titulaire: B. Braun Medical S.A.S., 92660 Boulogne Billancourt Cedex (FR)
(72) Inventeur: FORBER, Simon, 86240 Ligugé (FR)
(74) Mandataire: Winter, Brandl, Fürniss, Hübner, Röss, Kaiser, Polte - Partnerschaft mbB
(86) Numéro de dépôt international: PCT/EP2011/003162
(87) Numéro de publication internationale: WO 2012/000642

(56) Documents cités:
- EP-A2- 1 752 178
- WO-A1-2004/000408
- DE-A1-102007 011 303
- US-A1- 2003 105 431
- US-A1- 2004 186 434

## Description

La présente invention concerne une aiguille pour l'injection ou l'extraction de fluides avec un dispositif de sécurité pour la protection contre des blessures sur la pointe de l'aiguille.

Des aiguilles de différents types sont utilisées en médecine afin d'injecter des fluides dans le corps d'un patient ou d'en extraire. Il est commun à toutes les aiguilles qu'elles représentent un risque de blessure considérable pour le personnel traitant, notamment après l'utilisation lorsqu'elles sont contaminées de sang. Afin d'empêcher une infection, il est de ce fait important d'éviter des blessures lors du retrait de l'aiguille usagée.

Différents dispositifs de sécurité pour aiguilles ont été proposés dans le passé, lesquels doivent sécuriser l'aiguille et notamment la pointe dangereuse après le retrait. Le niveau de sécurité et le confort d'utilisation sont toutefois nettement insuffisants.

Le document FR 2 740 043 décrit un dispositif de protection d'une aiguille hypodermique comportant deux feuilles flexibles à l'extrémité proximale d'une aiguille qui sont d'abord disposées perpendiculairement à l'aiguille. Après la piqûre de l'aiguille, les feuilles se trouvent à la surface de la peau du patient. Lors du retrait de l'aiguille, une butée disposée au-dessus des feuilles est tenue et décalée vers le bas dans la direction distale de l'aiguille, moyennant quoi les feuilles sont amenées hors de leur position perpendiculaire à l'aiguille dans une position parallèle à l'aiguille. L'aiguille est ainsi entourée latéralement. Le risque de blessure est réduit.

Cette solution a toutefois des inconvénients décisifs. Le retrait de l'aiguille ne peut s'effectuer qu'avec une certaine force car les feuilles flexibles doivent être déformées sur une grande surface pour entourer l'aiguille. Dans la mesure où les feuilles viennent en appui avec la peau du patient dans leur position de départ, les feuilles se déplacent sur la peau lors du retrait de l'aiguille, ce qui peut conduire au pincement de peau ou à une action de force latérale sur l'aiguille. Un mécanisme supplémentaire doit par ailleurs sécuriser le dispositif de sécurité dans la position finale afin que la pointe de l'aiguille ne puisse pas en ressortir par glissement.

On connait du document US 6,224,569 81 un dispositif de sécurité qui siège dans sa position de départ a l'extrémité proximale de l'aiguille et peut être déplace sous la forme d'un manchon cylindrique le long de l'aiguille dans la direction distale. Deux ressorts se trouvent a l'intérieur du dispositif, lesquels sont maintenus par précontrainte contre la tige de l'aiguille. Si le dispositif est déplacé le long de la tige de l'aiguille dans la direction distale, l'extrémité distale du dispositif est poussée au-delà de la pointe de l'aiguille et la pointe pénètre à l'intérieur du dispositif. La tige de l'aiguille libère les ressorts qui se rabattent devant l'ouverture distale du dispositif. La pointe de l'aiguille est ainsi enfermée dans le dispositif.

On connait du document US 2003 / 0 105 431 A1 une aiguille ayant un dispositif de sécurité avec un clip à ressort qui applique une force latérale contre la tige de l'aiguille. La force nécessaire pour retirer l'aiguille et descendre le dispositif de sécurité vers la pointe est donc augmentée par le dispositif. Le clip s'active passivement dès que la pointe de l'aiguille l'a passé. L'élément d'activation est une partie de l'aiguille à diamètre élargi.

On connait aussi du document WO 2004 / 000 408 A1 une aiguille qui peut être retirée vers l'intérieur du dispositif qui reste ouvert du côté distal.

Un inconvénient de ce dispositif est toutefois qu'il ne peut être déplace que contre les ressorts précontraints le long de la tige de l'aiguille, ce qui nécessite une force relativement élevée. Une butée est en outre prévue afin que l'aiguille ne puisse pas être entièrement passée à travers le dispositif. Une butée sur la tige de l'aiguille ne peut toutefois être disposée que dans la région qui ne pénètre pas dans le tissu du patient. L'aiguille doit pour cela être de longueur conséquente et pouvoir être retirée suffisamment loin dans le dispositif de sécurité, ce qui suppose un dispositif de sécurité relativement long.

L'objet de la présente invention est de proposer un dispositif de sécurité pour une aiguille, notamment une aiguille médicale, qui évite les inconvénients décrits ci-dessus et présente notamment une construction plate qui rend l'aiguille facilement mobile et qui entoure sa pointe de manière sûre après activation du dispositif.

Cet objet est atteint selon l'invention par une aiguille avec une extrémité distale, une extrémité proximale et une tige s'étendant entre elles ainsi qu'une pointe à son extrémité distale. L'aiguille selon l'invention est pourvue d'un dispositif de sécurité pour la protection contre des blessures sur la pointe de l'aiguille, le dispositif de sécurité étant disposé de manière à pouvoir coulisser par glissement sur la tige entre une première position dans la région proximale de la tige et une seconde position à l'extrémité distale de la tige. Le dispositif de sécurité présente un logement fermé avec une ouverture proximale et une ouverture distale pour le passage de la tige de l'aiguille, la pointe de l'aiguille se trouvant à l'intérieur du logement lorsque le dispositif de sécurité se trouve dans la seconde position. Le dispositif de sécurité comprend un dispositif de fermeture qui ferme l'ouverture distale du logement lorsque la pointe de l'aiguille se trouve à l'intérieur du logement.

L'aiguille selon l'invention est caractérisée en ce que le dispositif de fermeture comprend au moins un élément de fermeture qui peut coulisser d'une première position à l'écart de l'ouverture distale dans une seconde position en face de l'ouverture. L'élément de fermeture coopère avec un élément d'activation qui est disposé coulissant sur la tige de l'aiguille. Un élément entraîneur qui limite la course de l'élément d'activation sur la tige de l'aiguille dans le sens distal, est en outre disposé sur la tige de l'aiguille. L'élément d'activation parvient lors du coulissement du dispositif de sécurité de sa première dans sa seconde position en butée avec l'élément entraîneur, moyennant quoi en cas de coulissement supplémentaire du dispositif de sécurité, l'élément d'activation déplace l'élément de fermeture de sa première dans sa seconde position et ferme ainsi l'ouverture distale dans le logement.

L'aiguille selon l'invention avec le dispositif de sécurité permet une utilisation sans danger de l'aiguille. Dans la première position du dispositif de sécurité, la pointe de l'aiguille est libérée, et l'aiguille est utilisable normalement. Etant donné qu'elle est encore neuve et inutilisée et généralement emballée de manière stérile, elle ne représente aucun risque particulier pour le personnel médical traitant. Même en cas de blessure avec la pointe de l'aiguille, il n'existe encore aucun risque d'infection.

L'aiguille est ensuite introduite dans le tissu d'un patient et utilisée conformément à l'usage. Lors du retrait de l'aiguille, le dispositif de sécurité est déplacé dans la direction distale vers la pointe de l'aiguille. Cela se produit d'abord sans résistance supplémentaire provoquée par le dispositif de sécurité. Vers la fin de la course, le ou les éléments de fermeture sont déplacés par butée de l'élément d'activation avec l'élément entraîneur de sa ou ses position(s) de départ dans une position en face de l'ouverture distale du logement dès que la pointe de l'aiguille est retirée dans l'intérieur du logement. Le logement est ainsi entièrement fermé. Une blessure du personnel traitant avec la pointe de l'aiguille contaminée est exclue.

Dans un mode de réalisation de l'invention, l'aiguille peut être une aiguille Huber qui dispose d'une extrémité coudée. La transition entre la section droite et la section coudée représente alors l'élément entraîneur.

Dans un autre mode de réalisation, l'élément entraîneur peut être un tronçon de la tige de l'aiguille ayant une section modifiée. La section de l'aiguille peut par exemple être localement ovale au lieu de circulaire. Les élément d'activation qui glissent sur la partie circulaire de la tige viennent en butée avec le tronçon de section ovale et déplacent ainsi les éléments de fermeture.

Les éléments de fermeture peuvent de préférence être reliés à l'élément d'activation par des bras, notamment par des bras déformables de manière non élastique. Il peut ainsi être garanti que les éléments de fermeture ne puissent plus être écartés involontairement de l'ouverture du logement après leur activation et que la pointe de l'aiguille puisse sortir du logement. Les forces nécessaires pour la déformation des bras sont en outre faibles de sorte que l'activation du dispositif de sécurité ne nécessite aucun effort particulier.

L'élément d'activation peut comprendre un manchon qui est disposé coulissant dans l'ouverture proximale du logement. Cela permet une structure particulièrement peu encombrante du dispositif de sécurité.

Le logement présente avantageusement une chambre dans la région du passage de l'aiguille dont la dimension convient à la réception de la section de l'aiguille entre élément entraîneur et pointe de l'aiguille. Cette chambre peut être cylindrique.

Le dispositif de sécurité de l'aiguille selon l'invention peut notamment présenter un ou deux éléments de fermeture.

De manière avantageuse, le logement peut présenter une butée qui empêche le déplacement de l'élément de fermeture de la seconde position. Cette butée peut notamment être agencée sur le fond du logement du dispositif de sécurité latéralement de l'ouverture distale. La butée empêche l'élément de fermeture, une fois arrivé dans sa seconde position devant l'ouverture, de quitter cette position.

Afin que l'aiguille puisse être manipulée facilement, elle peut être pourvue d'un socle dans la région proximale.

Etant donné que le dispositif de sécurité peut glisser sur la tige de l'aiguille, selon un mode de réalisation préféré, sans résistance au frottement sensible, une connexion amovible entre le socle et le dispositif de sécurité peut être prévue, notamment une connexion par encliquetage. Ainsi, un glissement précoce du dispositif de sécurité sur la tige de l'aiguille peut être évité. La connexion est de préférence facilement amovible de sorte qu'elle ne provoque aucun problème lors du maniement en cas de retrait de l'aiguille.

Un exemple de réalisation de l'invention est expliqué plus en détail dans la suite à l'aide des figures annexées :
- **La Figure 1**: illustre une aiguille selon l'invention avec un dispositif de sécurité en représentation éclatée ;
- **Les Figures 2a et 2b**: illustrent l'aiguille selon l'invention selon la Figure 1 en coupe transversale avec le dispositif de sécurité dans la première position, en agrandissement partiel à la Figure 2b ;
- **Les Figures 3a et 3b**: illustrent l'aiguille selon l'invention selon les Figure 1 et 2 en coupe transversale avec le dispositif de sécurité dans la seconde position, en agrandissement partiel à la Figure 3b ;
- **Les Figures 4a et 4b**: illustrent un autre mode de réalisation de l'invention, dans la première position à la Figure 4a et en agrandissement partiel dans la deuxième position à la Figure 4b.

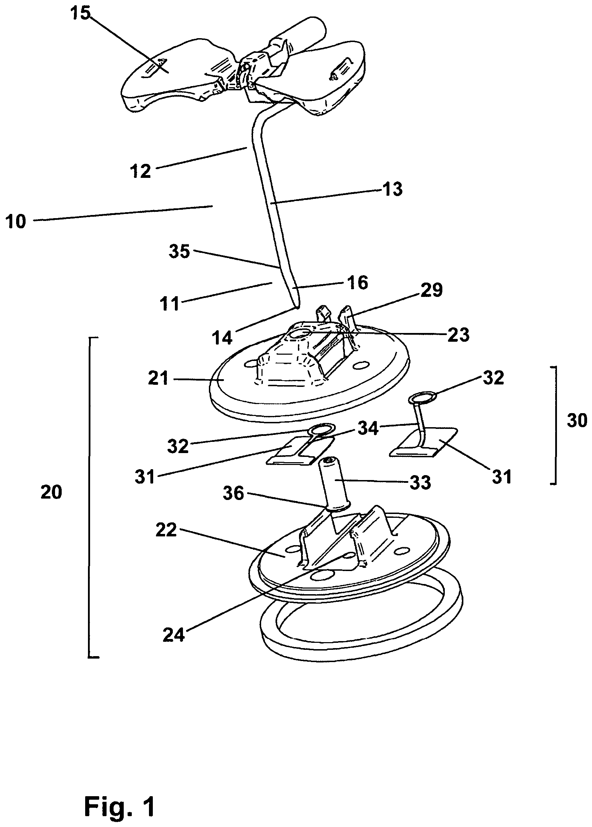

La **Figure 1** illustre une aiguille **10** selon l'invention avec un dispositif de sécurité **20** en représentation éclatée. Pour l'aiguille représentée, il s'agit d'une aiguille Huber en vue de l'utilisation pour des chambres implantables, pour la fourniture de médicaments à un patient. L'aiguille **10** présente une section d'aiguille coudée **16** sur la pointe **14** pour la ponction de la membrane d'une chambre implantable.

L'aiguille **10** dispose d'une extrémité distale **11** avec une pointe coudée **14** et d'une extrémité proximale **12** qui est pourvue d'un socle **15.**

L'aiguille **10** est en outre pourvue d'un dispositif de sécurité **20** qui est disposé coulissant sur la tige **13** de l'aiguille. Le dispositif de sécurité **20** peut y coulisser d'une première position à l'extrémité proximale **12** de la tige de l'aiguille **13** avant utilisation de l'aiguille dans une seconde position à l'extrémité distale **11** de l'aiguille **10** après utilisation, dans laquelle la pointe de l'aiguille **14** est sécurisée.

Le dispositif de sécurité **20** se compose d'un logement qui comprend un couvercle de logement **21** et un fond de logement **22.** Le couvercle de logement **21** présente une ouverture proximale **23** qui guide à glissement le logement sur la tige de l'aiguille **13.** Le fond du logement **22** présente une ouverture distale **24** du logement à travers laquelle l'aiguille peut traverser le logement.

Un élément de fermeture **31** est disposé à l'intérieur du logement **21, 22.** L'élément de fermeture est relié par un bras flexible **34** à un anneau **32** qui entoure de son côté un manchon **33** dont le col inférieur **36** présente un diamètre supérieur au diamètre interne de l'anneau **32.** Le manchon **33** glisse à l'état monté sur la tige de l'aiguille **13.** L'élément de fermeture **31,** le bras **34** et l'anneau **32** sont représentés à droite sur la Figure 1 à l'état activé lorsque le dispositif de sécurité **20** se trouve après l'utilisation dans la seconde position à l'extrémité distale **11** de l'aiguille **13.** La représentation à gauche montre l'élément de fermeture **31,** le bras **34** et l'anneau **32** dans la première position du dispositif de sécurité **20** avant l'utilisation de l'aiguille à l'extrémité proximale **12** de la tige de l'aiguille **13.**

Le dispositif de sécurité **20** se trouve dans la première position dans la région proximale **12** de la tige **13** près du socle **15.** La tige de l'aiguille **13** s'étend, vu dans le sens proximal vers distal, à travers l'ouverture proximale **23** du couvercle du logement **21,** à travers le manchon **33** et à travers l'ouverture distale du logement **24** dans le fond du logement **22** de sorte que la pointe de l'aiguille **14** est librement accessible et l'aiguille **10** peut être utilisée.

L'aiguille **10** peut ensuite être introduite dans le tissu d'un patient et utilisée conformément à l'usage. Un anneau **38** agencé sur le fond du logement **22** peut en l'occurrence servir de butée et vient alors en appui sur la peau du patient.

En vue de retirer l'aiguille, l'aiguille est saisie d'une main au niveau du socle **15** et le dispositif de sécurité **20** est maintenu en position par pression avec l'autre main sur le couvercle du logement **21** lors du retrait de l'aiguille **10.**

La connexion par encliquetage **29** qui sécurise le dispositif de sécurité **20** dans sa première position est détachée et la tige de l'aiguille **13** glisse alors à travers le dispositif de sécurité **20.** Aucune force de frottement sensible supplémentaire n'agit en l'occurrence entre la tige de l'aiguille **13** et le dispositif de sécurité **20** de sorte que l'aiguille peut être retirée sans problème du tissu d'un patient.

L'aiguille **10** peut glisser à travers le dispositif de sécurité **20** jusqu'à ce que le dispositif de sécurité **20** arrive à l'extrémité distale **11** de l'aiguille. Dès que le manchon **33** rencontre la transition **35** vers la région coudée **16** de l'aiguille, le manchon **33** ne peut plus continuer de glisser et est entraîné lors de la poursuite du mouvement de l'aiguille. Le manchon **33** entraîne en l'occurrence l'anneau **32** disposé sur son col inférieur **36,** lequel anneau est soulevé du manchon **33** lors de la poursuite du mouvement de l'aiguille. Le bras de connexion **34** appartenant à l'élément de fermeture **31** est en l'occurrence déformé de manière non élastique de sorte que l'élément de fermeture **31** est tiré vers l'intérieur et l'ouverture distale du logement **24** est fermée. Le couvercle du logement **21** et le fond du logement **22** sont réalisés à cet effet de telle sorte que l'élément de fermeture **31** peut se déplacer essentiellement parallèlement au fond **22.**

Le dispositif de fermeture **30** prend alors la forme illustrée à droite dans la figure 1.

La pointe de l'aiguille **14** est enfermée de manière sûre dans le logement **21, 22** dans la seconde position du dispositif de sécurité **20.** Le dispositif de fermeture **30** ne peut plus être ramené dans la position d'origine de par la déformation non élastique du bras **34** de sorte qu'une ouverture du dispositif par méprise et une sortie de la pointe de l'aiguille sont impossibles. Le dispositif de sécurité **20** ne peut également pas glisser de l'aiguille **10** dans la direction distale car la section d'aiguille coudée **16** ne peut pas glisser à travers le manchon **33** qui ne peut à son tour pas glisser de l'ouverture proximale **23** du couvercle du logement **21** en raison de son col inférieur élargi **36.**

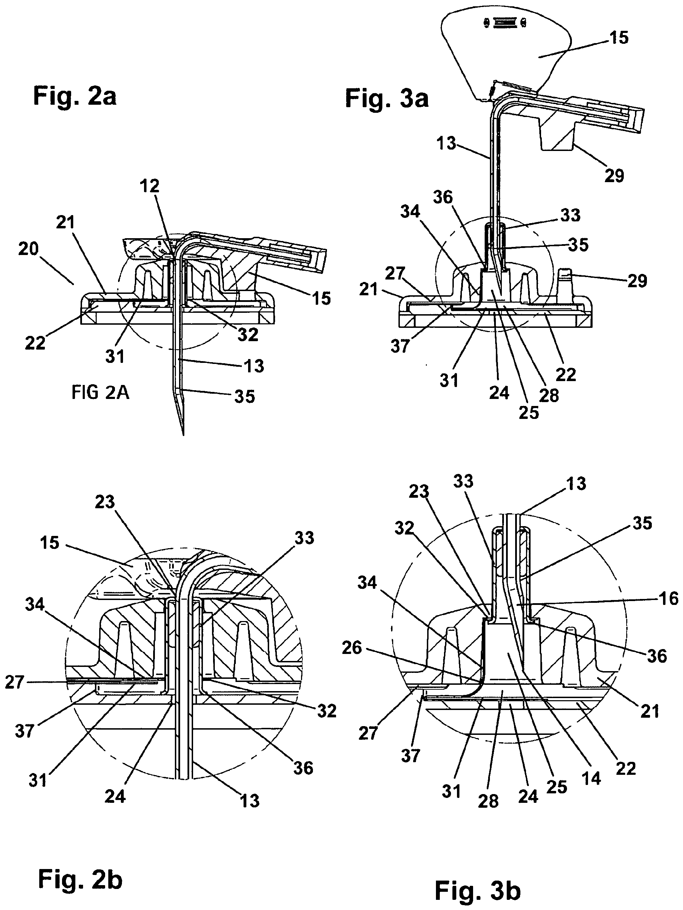

Les **Figures 2a** et **2b** illustrent l'aiguille avec le dispositif de sécurité **20** de la Figure 1 dans la première position avant son utilisation, en coupe transversale. La Figure 2b est en l'occurrence un agrandissement partiel de la Figure 2a.

Le dispositif de sécurité **20** se trouve dans la première position à l'extrémité proximale **12** de la tige de l'aiguille **13** en contact avec le socle **15.** L'aiguille traverse le logement **21, 22** du dispositif de sécurité à travers les ouvertures proximale et distale **23, 24.** Le socle **15** et le dispositif de sécurité **20** sont reliés ensemble par une connexion par encliquetage afin d'empêcher un glissement involontaire du dispositif de sécurité **20** en direction de l'extrémité distale **11** de l'aiguille **10.** La connexion par encliquetage est toutefois facilement amovible afin de garantir le retrait sans gêne de l'aiguille.

L'élément de fermeture **31** se trouve au sein du logement **21, 22** à côté de l'ouverture du logement **24** à travers laquelle la tige de l'aiguille **13** passe. On peut voir que l'élément de fermeture **31** est agencé dans une fente **27** entre le couvercle **21** et le fond **22** du logement un peut plus élevée par rapport au niveau du fond **22.**

Dans l'agrandissement de la Figure 2b, l'on reconnaît que l'élément de fermeture **31** est relié par le biais du bras **34** à un anneau **32** qui repose sur le manchon **33.** L'anneau **32** et le manchon **33** agissent comme élément d'activation pour l'élément de fermeture **31** du dispositif de sécurité lorsque le dispositif de sécurité est déplacé dans la seconde position.

Le manchon **33** est disposé coulissant dans l'ouverture proximale du logement **23** et guide la tige de l'aiguille **13.**

Pour l'activation, le dispositif de sécurité est déplacé dans la direction distale sur la tige de l'aiguille **13** lors du retrait de l'aiguille. La connexion par encliquetage est d'abord détachée entre socle **15** et dispositif de sécurité **20** de sorte que le dispositif de sécurité **20** peut glisser sur la tige de l'aiguille **13.** La tige de l'aiguille est en l'occurrence guidée à travers le manchon **33.**

Les **Figures 3a** et **3b** illustrent l'aiguille avec le dispositif de sécurité des Figures 1 et 2 dans la seconde position après son utilisation, en coupe transversale. La pointe de l'aiguille est alors sécurisée dans le dispositif. La Figure 3b est de nouveau un agrandissement partiel de la Figure 3a.

Si l'aiguille est retirée du tissu après l'utilisation, le socle **15** est à cet effet saisi d'une main tandis que l'autre main maintient le logement **21, 22** du dispositif de sécurité **20** en position. La connexion par encliquetage **29** entre socle **15** et dispositif de sécurité **20** est détachée et l'aiguille glisse à travers le dispositif de sécurité, étant guidée par le manchon **33.** Elle glisse jusqu'à sa section d'aiguille coudée **16** à travers le manchon **33.** La transition **35** vers la section coudée **16** de l'aiguille agit comme élément entraîneur. L'aiguille ne peut pas continuer de glisser à travers le manchon **33.** Le manchon est soulevé sur l'aiguille en cas de poursuite du coulissement et coulisse dans l'ouverture proximale **23** du couvercle du logement **21** vers le haut. L'anneau **32** est de ce fait également soulevé sur le col inférieur **36** du manchon **33.** Le bras flexible **34** se déforme de manière non élastique et tire l'élément de fermeture **31** vers l'intérieur devant l'ouverture distale **24** du dispositif de sécurité. Pendant son déplacement, l'élément de fermeture **31** sort de sa première position dans la fente **27,** pénètre de plus en plus dans le compartiment central **28** du logement et descend finalement la marche **37** entre la fente **27** et le compartiment central **28,** forcé par la déformation du bras flexible 34.

La pointe de l'aiguille **14** se trouve alors entièrement entourée à l'intérieur du logement **21, 22** et ne représente plus un danger. L'élément de fermeture **31** ne peut également plus être écarté de sa position devant l'ouverture **24** en raison de la déformation non élastique du bras **34** et car il vient en butée avec la marche **37** entre le compartiment central **28** du dispositif et la fente **27** ayant servi de logement pour l'élément de fermeture dans sa première position. En conséquence, l'aiguille ne peut plus sortir involontairement du logement.

L'on reconnaît sur les Figures 2b et 3b que la forme plate du logement **21, 22** avec la chambre cylindrique **25** apporte des avantages particuliers. Le logement **21, 22** est de construction plate et offre sur le côté supérieur suffisamment de surface pour le maintenir en position de manière sûre avec deux doigts tandis que l'aiguille est retirée. L'élément de fermeture **31** est en même temps maintenu dans une position parallèle au fond **22.** La chambre cylindrique **25** dans laquelle le manchon **33** se trouve dans la première position contribue à ce que le bras **34** soit déformé de manière non élastique, le mouvement de l'élément de fermeture **31** vers l'intérieur en direction de l'ouverture **24** étant renforcé par l'appui du bras **34** sur l'arête **26** de la chambre cylindrique **25.** La chambre cylindrique **25** présente suffisamment de hauteur pour recevoir la section d'aiguille coudée **16** sans pour autant agrandir la hauteur de construction du dispositif de sécurité dans les autres régions du logement.

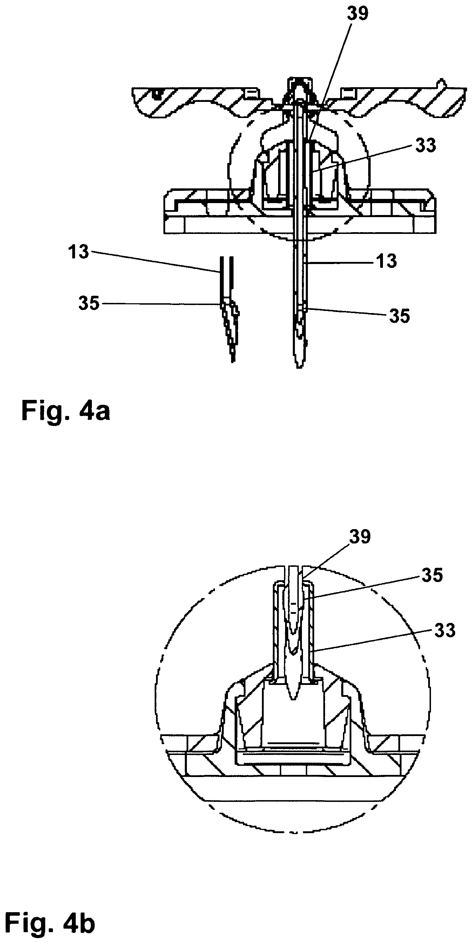

Les **Figures 4a** et **4b** illustrent un autre mode de réalisation de l'invention. La Figure 4a montre une aiguille dont la tige **13** a une section circulaire et qui présente un tronçon **35** de section modifiée. Le manchon **33** comporte une ouverture supérieure circulaire **39** adaptée à la section circulaire de la tige **13** de l'aiguille. Le tronçon de section modifiée **35** est disposé sur la tige **13** vers la fin de la course du dispositif de sécurité vers sa seconde position. Lors du glissement du dispositif sur la tige **13** de la première vers la seconde position, l'ouverture **39** du manchon **33** vient en butée avec le tronçon de section modifiée **35** comme représenté en agrandissement partiel dans la Figure 4b. Le tronçon de section modifiée **35** représente l'élément entraîneur. Les éléments de fermeture qui ne sont pas représentés dans cette figure sont ensuite activés de la même manière comme décrit ci-dessus pour le mode de réalisation des Figures 1 à 3.

## Revendications

1. Aiguille (10)
avec une extrémité distale (11), une extrémité proximale (12) et une tige (13) s'étendant entre elles ainsi qu'une pointe (14) à son extrémité distale (11),
pourvue d'un dispositif de sécurité (20) pour la protection contre des blessures sur la pointe de l'aiguille (14),
le dispositif de sécurité (20) étant disposé de manière à pouvoir coulisser par glissement sur la tige (13) entre une première position dans la région proximale (12) de la tige (13) et une seconde position à l'extrémité distale (11) de la tige (13)
et présentant un logement fermé (21, 22) avec une ouverture proximale (23) et une ouverture distale (24) pour le passage de la tige de l'aiguille (13),
la pointe de l'aiguille (14) se trouvant à l'intérieur du logement (21, 22) lorsque le dispositif de sécurité (20) se trouve dans la seconde position,
et le dispositif de sécurité (20) comprenant un dispositif de fermeture (30) qui ferme l'ouverture distale (24) du logement (21, 22) lorsque la pointe de l'aiguille (14) se trouve à l'intérieur du logement (21, 22),
**caractérisée en ce que**
le dispositif de fermeture (30) comprend au moins un élément de fermeture (31) qui peut coulisser d'une première position à l'écart de l'ouverture (24) dans une seconde position en face de l'ouverture (24),
et coopère avec un élément d'activation (32, 33) qui est disposé coulissant sur la tige de l'aiguille (13),
et qu'un élément entraîneur (35) qui limite la course de l'élément d'activation (32, 33) sur la tige de l'aiguille (13) dans le sens distal, est disposé sur la tige de l'aiguille (13),
et que l'élément d'activation (32, 33) parvient lors du coulissement du dispositif de sécurité (20) de sa première dans sa seconde position en butée avec l'élément entraîneur (35), moyennant quoi en cas de coulissement supplémentaire du dispositif de sécurité (20), l'élément d'activation (32, 33) déplace l'élément de fermeture (31) de sa première dans sa seconde position et ferme ainsi l'ouverture (24) dans le logement (21, 22).

2. Aiguille (10) selon la revendication 1, **caractérisée en ce qu'**une modification de la section de la tige (13) de l'aiguille (10) représente l'élément entraîneur (35).

3. Aiguille (10) selon la revendication 1, **caractérisée en ce que** l'aiguille (10) est une aiguille Huber et la transition entre la section droite et la section coudée (16) de l'aiguille (10) représente l'élément entraîneur (35).

4. Aiguille (10) selon l'une quelconque des revendications 1 à 3, **caractérisée en ce que** les éléments de fermeture (31) sont reliés à l'élément d'activation (32, 33) par des bras (34), notamment par des bras déformables de manière non élastique.

5. Aiguille (10) selon l'une quelconque des revendications 1 à 4, **caractérisée en ce que** l'élément d'activation comprend un manchon (33) qui est disposé coulissant dans l'ouverture proximale (23) du logement.

6. Aiguille (10) selon l'une quelconque des revendications 1 à 5, **caractérisée en ce que** le logement (21) présente une chambre (25) dans la région du passage de l'aiguille dont la dimension convient à la réception de la section de l'aiguille (16) entre élément entraîneur (35) et pointe de l'aiguille (14).

7. Aiguille (10) selon l'une quelconque des revendications 1 à 6, **caractérisée en ce que** le dispositif de sécurité (20) présente un ou deux éléments de fermeture (31).

8. Aiguille (10) selon l'une quelconque des revendications 1 à 7, **caractérisée en ce que** le logement (21, 22) présente une butée (37) qui empêche le déplacement de l'élément de fermeture (31) de la seconde position.

9. Aiguille (10) selon l'une quelconque des revendications 1 à 8, **caractérisée en ce que** l'aiguille (10) présente un socle (15) dans la région proximale (12).

10. Aiguille (10) selon la revendication 9, **caractérisée en ce que** le socle (15) et le dispositif de sécurité (20) sont reliés ensemble de manière amovible dans la première position, notamment par une connexion par encliquetage (29).

## Patentansprüche

1. Nadel (10)
mit einem distalen Ende (11), einem proximalen Ende (12) und einem sich zwischen ihnen erstreckenden Schaft (13) sowie einer Spitze (14) an ihrem distalen Ende (11),
ausgestattet mit einer Sicherheitsvorrichtung (20) zum Schutz gegen Verletzungen an der Spitze der Nadel (14),
wobei die Sicherheitsvorrichtung (20) so angebracht ist, dass sie durch Gleiten auf dem Schaft (13) zwischen einer ersten Position im proximalen Bereich (12) des Schafts (13) und einer zweiten Position am distalen Ende (11) des Schafts (13) verschoben werden kann
und mit einem geschlossenen Gehäuse (21, 22) mit einer proximalen Öffnung (23) und einer distalen Öffnung (24) zum Durchgang des Schafts der Nadel (13),
wobei sich die Spitze der Nadel (14) im Gehäuse (21, 22) befindet, wenn sich die Sicherheitsvorrichtung (20) in der zweiten Position befindet,
und die Sicherheitsvorrichtung (20) eine Verschlussvorrichtung (30) umfasst, die die distale Öffnung (24) des Gehäuses (21, 22) schließt, wenn sich die Spitze der Nadel (14) im Gehäuse (21, 22) befindet,
**dadurch gekennzeichnet, dass**
die Verschlussvorrichtung (30) mindestens ein Verschlusselement (31) umfasst, das von einer ersten Position abseits der Öffnung (24) in eine zweite Position gegenüber der Öffnung (24) gleiten kann,
und mit einem Aktivierungselement (32, 33) kooperiert, das verschiebbar auf dem Schaft der Nadel (13) angebracht ist,
und dass ein Mitnehmerelement (35), das den Weg des Aktivierungselements (32, 33) auf dem Schaft der Nadel (13) in distaler Richtung begrenzt, auf dem Schaft der Nadel (13) angebracht ist,
und dass das Aktivierungselement (32, 33) beim Verschieben der Sicherheitsvorrichtung (20) von seiner ersten in seine zweite Position am Endanschlag mit dem Mitnehmerelement (35) gelangt, wodurch das Aktivierungselement (32, 33) bei zusätzlichem Verschieben der Sicherheitsvorrichtung (20) das Verschlusselement (31) von seiner ersten in seine zweite Position bringt und so die Öffnung (24) im Gehäuse (21, 22) schließt.

2. Nadel (10) nach Anspruch 1, **dadurch gekennzeichnet, dass** eine Änderung des Abschnitts des Schafts (13) der Nadel (10) das Mitnehmerelement (35) darstellt.

3. Nadel (10) nach Anspruch 1, **dadurch gekennzeichnet, dass** die Nadel (10) eine Huber-Nadel ist und der Übergang zwischen dem geraden Abschnitt und dem winkelförmigen Abschnitt (16) der Nadel (10) das Mitnehmerelement (35) darstellt.

4. Nadel (10) nach einem der Ansprüche 1 bis 3, **dadurch gekennzeichnet, dass** die Verschlusselemente (31) über Arme (34) mit dem Aktivierungselement (32, 33) verbunden sind, insbesondere durch Arme, die auf nicht elastische Art verformbar sind.

5. Nadel (10) nach einem der Ansprüche 1 bis 4, **dadurch gekennzeichnet, dass** das Aktivierungselement eine Muffe (33) umfasst, die verschiebbar in der proximalen Öffnung (23) des Gehäuses angebracht ist.

6. Nadel (10) nach einem der Ansprüche 1 bis 5, **dadurch gekennzeichnet, dass** das Gehäuse (21) eine Kammer (25) im Bereich des Durchgangs der Nadel aufweist, deren Abmessungen die Aufnahme des Nadelquerschnitts (16) zwischen Mitnehmerelement (35) und Spitze der Nadel (14) ermöglicht.

7. Nadel (10) nach einem der Ansprüche 1 bis 6, **dadurch gekennzeichnet, dass** die Sicherheitsvorrichtung (20) ein oder zwei Verschlusselemente (31) aufweist.

8. Nadel (10) nach einem der Ansprüche 1 bis 7, **dadurch gekennzeichnet, dass** das Gehäuse (21, 22) einen Endanschlag (37) aufweist, der eine Verschiebung des Verschlusselements (31) aus der zweiten Position verhindert.

9. Nadel (10) nach einem der Ansprüche 1 bis 8, **dadurch gekennzeichnet, dass** die Nadel (10) einen Sockel (15) im proximalen Bereich (12) aufweist.

10. Nadel (10) nach Anspruch 9, **dadurch gekennzeichnet, dass** der Sockel (15) und die Sicherheitsvorrichtung (20) abnehmbar miteinander in der ersten Position verbunden sind, insbesondere durch eine Einrastverbindung (29).

## Claims

1. Needle (10)
with a distal end (11), a proximal end (12) and a shank (13) extending between them as well as a tip (14) at its distal end (11),
provided with a safety device (20) for protection against injuries on the tip of the needle (14),
the safety device (20) being arranged so as to be able to slide by sliding on the shank (13) between a first position in the proximal area (12) of the shank (13) and a second position at the distal end (11) of the shank (13)
and having a closed housing (21, 22) with a proximal opening (23) and a distal opening (24) for passing the shank of the needle (13),
the tip of the needle (14) being found inside the housing (21, 22) when the safety device (20) is found in the second position,
and the safety device (20) comprising a closing device (30) which closes the distal opening (24) of the housing (21, 22) when the tip of the needle (14) is found inside the housing (21, 22),
**characterised in that**
the closing device (30) comprises at least one closing member (31) which can slide from a first position away from the opening (24) in a second position opposite the opening (24),
and cooperates with an activation member (32, 33) which is arranged to slide on the shank of the needle (13),
and that a drive member (35) which limits the travel of the activation member (32, 33) on the shank of the needle (13) in the distal direction, is arranged on the shank of the needle (13),
and that the activation member (32, 33), during the sliding of the safety device (20) from its first to its second position, comes up against the drive member (35), by means of which in the case of additional sliding of the safety device (20), the activation member (32, 33) displaces the closing member (31) from its first to its second position and thus closes the opening (24) in the housing (21, 22).

2. Needle (10) according to claim 1, **characterised in that** a modification of the cross-section of the shank (13) of the needle (10) represents the drive member (35).

3. Needle (10) according to claim 1, **characterised in that** the needle (10) is a Huber needle and the transition between the straight section and the bent section (16) of the needle (10) represents the drive member (35).

4. Needle (10) according to any one of claims 1 to 3, **characterised in that** the closing members (31) are connected to the activation member (32, 33) by arms (34), in particular by arms that can deform in a non-elastic manner.

5. Needle (10) according to any one of claims 1 to 4, **characterised in that** the activation member comprises a sleeve (33) which is arranged to slide in the proximal opening (23) of the housing.

6. Needle (10) according to any one of claims 1 to 5, **characterised in that** the housing (21) has a chamber (25) in the area of the passing of the needle of which the dimension is suited to receive the section of the needle (16) between the drive member (35) and the tip of the needle (14).

7. Needle (10) according to any one of claims 1 to 6, **characterised in that** the safety device (20) has one or two closing members (31).

8. Needle (10) according to any one of claims 1 to 7, **characterised in that** the housing (21, 22) has an end stop (37) which prevents the displacement of the closing member (31) from the second position.

9. Needle (10) according to any one of claims 1 to 8, **characterised in that** the needle (10) has a base (15) in the proximal area (12).

10. Needle (10) according to claim 9, **characterised in that** the base (15) and the safety device (20) are connected together in a removable manner in the first position, in particular by a snap-fitting connection (29).
